# EUROPEAN PATENT APPLICATION

(11) **EP 1 321 478 A2**
(43) Date of publication of application: **25.06.2003**
(21) Application number: 02258743.0
(22) Date of filing: 18.12.2002
(51) Int. Cl.: C07K 16/12, A61K 39/09, G01N 33/569, G01N 33/577

(54) **Antibody against Streptococcus mutans and its use in an immunoassay**

(30) Priority: 18.12.2001 JP 2001384595
(71) Applicant: Tokuyama Dental Corporation, Tokyo 110-0016 (JP); Nihon University, School Juridical Person, Tokyo 102-8275 (JP)
(72) Inventor: Ukaji, Fumio, c/o Tokuyama Dental Corporation, Tokyo 100-0016 (JP); Hirata, Kouichirou, c/o Tokuyama Dental Corp., Tokyo 100-0016 (JP); Hanyu, Naohiro, c/o Tokuyama Dental Corporation, Tokyo 100-0016 (JP); Fukushima, Kazuo, c/o Nihon University, Chiyoda-ku, Tokyo 102-8275 (JP)
(74) Representative: Maschio, Antonio

(57) **Abstract**

*Streptococcus mutans* in saliva or dental plaque collected from the oral cavity can be measured by the method of the present invention conveniently with high sensitivity without the performance of any complex procedure, such as culturing. The reactivity of the antibody of the present invention against *Streptococcus mutans* is 1000-fold or more greater than its reactivity against each of *Streptococcus gordonii, Streptococcus salivarius, Streptococcus oralis, Streptococcus mitis,* and *Streptococcus sanguis*.

## Description

### FIELD OF THE INVENTION

The present invention relates to a polyclonal antibody which is specific for *Streptococcus mutans*, a method for producing the polyclonal antibody, a method for measuring a level of *Streptococcus mutans*, an immunoassay reagent and an immunochromatography test strip.

### BACKGROUND OF THE INVENTION

Generally, a group of lactic acid producing bacteria referred to as mutans streptococci is known to be deeply associated with the onset of dental caries.

The mutans streptococci is classified into 7 species which are serologically and genetically different: *Streptococcus cricetus* (*S. cricetus,* serotype a), *Streptococcus rattus (S. rattus,* serotype b), *Streptococcus mutans* (*S. mutans,* serotype c, e and f), *Streptococcus ferus* (*S. ferus*, serotype c), *Streptococcus macacae* (*S. macacae,* serotype c), *Streptococcus sobrinus* (*S. sobrinus*, serotype d and g) and *Streptococcus downey* (*S. downey*, serotype h).

It is reported that a concentration of 10⁵ to 10⁶ cells/ml of these mutans streptococci in saliva suggests the risk of dental caries, and a concentration of 10⁶ cells/ml or more suggests a particular risk of dental caries. Knowing the cell count of mutans streptococci which are present in human oral cavity makes it possible to determine the degree of individual risk of dental caries. The concentration of mutans streptococci is generally measured by culturing mutans streptococci in saliva using selective media supplemented with bacitracin and quantifying colonies (measurement kits for this purpose are marketed). The concentration of each species of mutans streptococci in saliva can be roughly determined by culturing in a similar manner, identifying colonies of each species of mutans streptococci in saliva from the obtained colonies, and examining the number of the colonies. In addition, known methods of identification include a biochemical method, such as a sugar fermentation test, a genetic method using DNA probes, and an immunological method or the like using serotype-specific antibodies.

In recent years, it became clear that mutans streptococci that are mainly present in human oral cavity are 2 strains: *Streptococcus mutans* and *Streptococcus sobrinus.* Particularly, *Streptococcus mutans* is isolated from human oral cavity at high frequency (it is isolated from 90% or more subjects), suggesting its deep association with the incidence of dental caries.

### SUMMARY OF THE INVENTION

A culture method is now generally executed as a method for measuring oral mutans streptococci. However, the culture method requires culturing procedures and skilled manipulation for identifying a strain type based on the morphology of an isolated colony. Thus, the culture method has a problem in terms of testing time and the complexity of the procedure.

As a method for directly detecting *Streptococcus mutans* from clinical samples, such as dental plaque or saliva, immunoassays using various monoclonal antibodies or polyclonal antibodies have been reported. These methods are advantageous in that they need no culturing and the time required for detection can be greatly shortened.

It is known that various Streptococcal species inhabit naturally in human oral cavity. The abundance ratio of *Streptococcus* in bacterial flora differs depending on the oral site. It is known that in dental plaque and saliva which are used for determination of the risk of dental caries, *Streptococcus* account for about 50% of the bacterial flora (edited by Hidehiko MUKASA, Molecular Biology of Cariogenic Bacteria, 29-37, 1st edition, Quintescence Publishing Co., Ltd. 1997), and it is said that the total bacterial count in 1 ml of saliva is 10⁸ cells/ml or more. Since the number of mutans streptococci is generally 10⁷ cells/ml or less, it can be said that most of oral streptococci are those other than mutans streptococci. Among streptococci other than mutans streptococci, *Streptococcus gordonii, Streptococcus salivarius, Streptococcus oralis, Streptococcus mitis* and *Streptococcus sanguis* are the major bacteria in oral bacterial flora. Each of these bacteria is usually present in the order of 10⁷ cells/ml in saliva, and the cell count can reach the order of about 10⁸ cells/ml in some cases.

Therefore, to determine the risk of dental caries, *Streptococcus mutans* in the order of 10⁵ cells/ml co-existing with oral streptococci other than mutans streptococci in the order of 10⁸ cells/ml is measured. Specifically, even when the cell count of oral streptococci other than mutans streptococci is about 1000-fold greater than that of *Streptococcus mutans, Streptococcus mutans* should still be measured accurately. This measurement needs an antibody having a high antibody titer and a remarkably high specificity. Such an antibody has significantly high reactivity against 10⁵ cells/ml of oral *Streptococcus mutans* compared to its reactivity against 10⁸ cells/ml of oral streptococci other than mutans streptococci.

A method using a monoclonal antibody that has been proposed involves mixing a monoclonal antibody against enzyme-labeled *Streptococcus mutans* with a sample, filtering the mixture using a membrane filter with a pore diameter of 0.45 µm to retain only the enzyme-labeled antibody bound to *Streptococcus mutans* on the filter, and detecting the target by coloration after addition of an enzyme substrate (Yutaka YASUTOMI et al., The Japanese Journal of Pediatric Dentistry, 30:186-193, 1992, Japanese Patent No. 3093833). However, the monoclonal antibody used in this method has reactivity against *Streptococcus mutans* at a level of 10⁵ cells/ml which is of the same degree as its cross-reactivity against *Streptococcus salivarius* at a level of 10⁷ cells/ml. *Streptococcus salivarius* may exist in oral cavity at a level of 10⁷ to 10⁸ cells/ml. In this case, there is a problem that *Streptococcus mutans* cannot be measured accurately.

A measurement method noticing reactivity against oral streptococci other than mutans streptococci proposed in JP Publication (Unexamined Application) No. 10-36400 uses a monoclonal antibody against *Streptococcus mutans*, which does not react with *Streptococcus mitis, Streptococcus salivarius* and *Streptococcus sanguis.* The specificity of the monoclonal antibody has been evaluated by examining the reactivity of an antibody against the above 3 strains and cultured cells of *Streptococcus mutans*, which are at a fixed level (10⁸ cells/ml), by double dilution series of the antibody. Thus, a property disclosed therein is that even in the range of an excess amount of antibody wherein the dose-response curve of antigen-antibody reaction is at a plateau, no cross-reactivity is detected against *Streptococcus mitis, Streptococcus salivarius* or *Streptococcus sanguis.* However, reactivity against 10⁵ cells/ml of *Streptococcus mutans* is not yet examined. Hence, it is unknown whether *Streptococcus mutans* can be accurately measured when oral streptococci other than mutans streptococci exist in a number about 1000-fold greater than that of *Streptococcus mutans*. Another problem is that reactivities against other species of oral streptococci including *Streptococcus gordonii* and *Streptococcus oralis* have not been studied at all.

In addition, a measurement method proposed in JP Publication (Unexamined Application) No. 2001-172299 uses a monoclonal antibody which does not react with *Streptococcus mitis, Streptococcus salivarius, Streptococcus sanguis* and *Porphyromonas gingivalis*, but reacts with *Streptococcus mutans* and *Streptococcus sobrinus*. The properties of the monoclonal antibody disclosed therein are such that when the reactivity against a fixed amount of cultured cells (dry weight: 5 µg) is examined, no cross-reactivity can be detected against *Streptococcus sanguis, Streptococcus salivarius, Streptococcus mitis, Streptococcus rattus, Streptococcus ferus, Streptococcus anginosus, Lactobacillus casei* and *Porphyromonas gingivalis*, and that although 10⁵ cells/ml of *Streptococcus mutans* can be measured, no cross-reactivity can be detected against 10⁶ cells/ml of *Streptocccus salivarius.* Problems in this case are again that it is unknown whether *Streptococcus mutans* can be accurately measured when oral streptococci other than mutans streptococci exist in a number about 1000-fold greater than that of *Streptococcus mutans*, and that reactivities against other species of oral streptococci including *Streptococcus gordonii* and *Streptococcus oralis* have not been studied at all.

On the other hand, as a method using a polyclonal antibody against *Streptococcus mutans*, a method using a latex agglutination method on a glass slide has been reported (Tsutomu TAKEI, Journal of Osaka University Dental Society, 35:93-109, 1990; Takei, T. Archs. oral. Biol. 37: 99-104, 1992; JP Publication (Unexamined Application) No. 1-250067). It is generally known to be difficult to obtain polyclonal antibodies with high specificity even when an antibody which causes cross-reaction is removed by affinity purification, because the antigenicity of *Streptococcus mutans* is not so high (edited by Shigeyuki HAMADA, Advances in Basic Cariology and Periodontology, Vol. 1, 17-23, 1st edition, Nippon Dental Review Publishing Co., Ltd. 1982). This method uses a polyclonal antibody prepared by immunizing a rabbit with the cells of *Streptococcus mutans*, but not subjected to affinity-purification. Therefore, the specificity of a polyclonal antibody used in this method may be low. Hence, with this method, positive reaction was obtained only for a cell sample solution at a concentration of 1 x 10⁶ cells/ml or more, and the sensitivity achieved by this method is insufficient.

Furthermore, an antibody prepared by treating a polyclonal antibody against *Streptococcus mutans* to have reduced cross-reactivity against other bacteria (hereinafter, also referred to as "treated polyclonal antibody") is known as a serotype-specific polyclonal antibody . The serotype-specific polyclonal antibody is produced by noticing each cross reaction among each serotype of mutans streptococci, and performing absorption treatment using mutans streptococci having a serotype other than that used for immunization.

For example, Bratthall obtained a serotype c- and e-specific antibody by performing absorption treatment using streptococci of serotype a or b on antibodies provided by immunizing animals with standard cells of *Streptococcus mutans* of serotype c (Bratthall, D., Odont. Revy. 23: 181-196, 1972). In addition, Ota obtained specific antibodies by respectively performing absorption treatment on antibodies provided by immunizing with standard cells of *Streptococcus mutans* of serotype c, e or f, using bacteria of serotype a, d, e and f in the case of anti-c antibody; using bacteria of serotype c, f and g in the case of anti-e antibody; or using bacteria of serotype e in the case of anti-f antibody (Ota, F, Zbl. Bakt. Hyg. A265: 330-339, 1987).

However, regarding the polyclonal antibodies that have been so far obtained including the above polyclonal antibodies prepared by Bratthal and Ota, cross-reactivity against oral streptococci other than mutans streptococci, such as *Streptococcus gordonii, Streptococcus salivarius, Streptococcus oralis, Streptococcus mitis, Streptococcus sanguis* or the like has not been sufficiently studied.

Further, a measurement method using a polyclonal antibody known as a fluorescent antibody technique (Walter, J., J. Dent. Res. 55: A87-A93, 1976, Babaahmady, K. G., Caries Res. 32: 51-58, 1998) has a problem in that it requires a complicated maneuver involving counting cells with a relatively high fluorescence intensity while visually determining the fluorescence intensity detected with a fluorescence microscope.

Regarding measurement of *Streptococcus mutans* in clinical samples directly prepared from saliva or dental plaque, there is a need to develop a convenient measurement method with a lower detection limit of 10⁵ cells/ml for determination of the risk of dental caries. However, no known method for measuring *Streptococcus mutans* satisfies the requirements.

Therefore, an object of the present invention is to provide a polyclonal antibody, a method for producing the polyclonal antibody, a method for measuring *Streptococcus mutans,* an immunoassay reagent and a immunochromatography test strip, which enable convenient and highly sensitive measurement of *Streptococcus mutans* in saliva or dental plaque collected from oral cavity without performing complicated procedures, such as culturing.

As a result of considerable effort in view of the above problems, we have completed the present invention by succeeding in creating a polyclonal antibody which is capable of controlling cross-reactivity against each species existing in a test solution including *Streptococcus gordonii, Streptococcus salivarious, Streptococcus oralis, Streptococcus mitis* and *Streptococcus sanguis* at a sufficiently low level, and thus is capable of specifically detecting and quantitatively determining *Streptococcus mutans.*

More particularly, the present invention encompasses the following:
(1) A polyclonal antibody, whose reactivity against *Streptococcus mutans* is 1000-fold or more greater than its reactivity against each of *Streptococcus gordonii, Streptococcus salivarius, Streptococcus oralis, Streptococcus mitis* and *Streptococcus sanguis.*
(2) The polyclonal antibody of (1), which is obtained by immunizing an immune animal with the whole cells of *Streptococcus mutans* treated with protease, or the antigen extract thereof.
(3) The polyclonal antibody of (1), which is purified by affinity purification using the whole cells of *Streptococcus mutans* treated with protease, or the antigen extract thereof.
(4) A method for producing a polyclonal antibody of (1) which is obtained by immunizing an immune animal with the whole cells of *Streptococcus mutans* treated with protease, or the antigen extract thereof.
(5) A method for producing a polyclonal antibody of (1), which comprises obtaining antibodies as raw material by immunizing an immune animal with at least one type of substance selected from the group consisting of the whole cells of *Streptococcus mutans* treated with protease, the antigen extract thereof, the whole cells of *Streptococcus mutans* and the antigen extract thereof; and then purifying from the antibodies as raw material by affinity purification using the whole cells of *Streptococcus mutans* treated with protease or the antigen extract thereof.
(6) A method for measuring *Streptococcus mutans*, which comprises measuring *Streptococcus mutans* contained in a test solution by an immunoassay using the polyclonal antibody of any one of (1) to (3).
(7) The method for measuring *Streptococcus mutans* of (6), wherein the immunoassay is an immunochromatography method.
(8) The method for measuring the antigen extract obtained from *Streptococcus mutans*, which comprises measuring the antigen extract obtained from *Streptococcus mutans* contained in a test solution by an immunoassay using the polyclonal antibody of any one of (1) to (3).
(9) An immunoassay reagent, which comprises the polyclonal antibody of any one of (1) to (3).
(10) An immunochromatography test strip, which comprises: a sample pad for absorbing and retaining temporarily a test solution, a conjugate pad for retaining temporarily a labeled antibody, and a development membrane wherein antibodies for detection are immobilized and the test solution temporarily absorbed and retained by the above sample pad and the labeled antibodies that flow from the above conjugate pad accompanying the test solution are spread:
   and wherein these components are joined in this order, and one of or both the above labeled antibody and the antibody for detection are the polyclonal antibodies of any one of (1) to (3).

According to the polyclonal antibody of the present invention, *Streptococcus mutans* existing in a test solution at a concentration of about 10⁵ cells/ml can be detected with high sensitivity and quantitatively determined. In addition, when the polyclonal antibody according to the present invention is used, an immunoassay is applied, and *Streptococcus mutans* can be measured without culturing *Streptococcus mutans* in saliva or dental plaque. Such a method has been made possible because we have clarified the properties of a polyclonal antibody required for highly sensitive detection of *Streptococcus mutans* and the polyclonal antibody according to the present invention satisfies these properties.

Further, according to the method for producing the polyclonal antibody according to the present invention, immunization is performed using cells from which cellular surface proteins present on the cell surface of *Streptococcus mutans* that are thought to cause cross-reaction have been removed by treatment with protease (hereinafter, also referred to as "protease-treated cells"), so that production of an antibody causing cross-reaction can be controlled.

Furthermore, in the method for producing a polyclonal antibody according to the present invention, purification is preferably performed by an affinity purification method using the protease-treated whole cells of *Streptococcus mutans* or the antigen extract thereof. The polyclonal antibody that can be obtained in this case has an antibody constituent bound to the above protease-treated whole cells of *Streptococcus mutans*, or the antigen extract thereof. Further, the polyclonal antibody having an improved specificity against *Streptococcus mutans* can be produced.

### BRIEF DESCRIPTION OF THE DRAWINGS

Figure 1 is a schematic diagram showing each member of a strip to be used in the immunochromatography method of the present invention.
Figure 2 is a side view showing the strip to be used in the immunochromatography method of the present invention.
Explanation for numerals:
1... strip
2... sample pad
3... conjugate pad
4... development membrane
5... absorbent pad
6... detection line
7... control determination line

### DETAILED DESCRIPTION OF THE PREFERRED EMBODIMENTS

The embodiments of the present invention are hereafter described in detail.

### 1. Polyclonal antibody according to the present invention

The ratio of the reactivity of the polyclonal antibody according to the present invention against *Streptococcus mutans* to its reactivity against each of *Streptococcus gordonii, Streptococcus salivarius, Streptococcus oralis, Streptococcus mitis* and *Streptococcus sanguis* stands at 1000 or more to 1. In other words, the polyclonal antibody according to the present invention is characterized in that the quotient (hereinafter, this may also be referred to as "M/S reactivity multiplication factor") given when the reactivity against *Streptococcus mutans* is divided by its reactivity against *Streptococcus gordonii, Streptococcus salivarius, Streptococcus oralis, Streptococcus mitis* or *Streptococcus sanguis* is always 1000 or more.

The term *"Streptococcus mutans"* means a streptococcus which is serotyped as c, e or f among a group of mutans streptococci. Examples of a standard strain of serotype c include strains of Ingbritt, MT6R and the like, and examples of a standard strain of serotype e include strains of LM7, P4 and the like, and examples of a standard strain of serotype f include strains of SE11, OMZ175 and the like.

Further, examples of a standard strain of *Streptococcus gordonii* include strains of Challis, ATCC10558 and the like; that of *Streptococcus salivarius* include strains of ATCC9222, IFO13956 and the like; that of *Streptococcus oralis* include strains of ATCC10557, ATCC35037 and the like; that of *Streptococcus mitis* include strains of ATCC15910, ATCC15911 and the like; and that of *Streptococcus sanguis* include strains of ATCC10556, ATCC10557 and the like.

The term "polyclonal antibody" (hereinafter, also simply referred to as "antibody") means an antibody fraction contained in antiserum. The globulin class of the antibody is not limited, and currently known antibodies of any globulin class may be included. In addition, the meaning of the polyclonal antibody includes not only whole antibody molecules, but also partially decomposed products of the antibody (Fab, Fab', Fab' 2 and the like), and a partial structure and the like in which active fragments (the antigen recognition site of an antibody) of the antibody are present.

Furthermore, the phrase "M/S reactivity multiplication factor of the antibody is 1000 or more" means that when antigen-antibody reaction is detected by a standard known immunoassay, such as enzyme-linked immunosorbent assay (hereinafter, this may also be referred to as "ELISA method") or radioimmunoassay, using as an antigen *Streptococcus mutans, Streptococcus gordonii, Streptococcus salivarius, Streptococcus oralis, Streptococcus mitis* or *Streptococcus sanguis*, reaction detected when *Streptococcus mutans* is used as an antigen is 1000-fold or more greater than that detected when *Streptococcus gordonii, Streptococcus salivarius, Streptococcus oralis, Streptococcus mitis* or *Streptococcus sanguis* (hereinafter, these streptococci are together also referred to as "other streptococci") is used as an antigen.

The M/S reactivity multiplication factor of the antibody can be obtained by comparison of the amounts of antigen (bacterial cell number) at which the same reactivity is detected, when *Streptococcus mutans* and other streptococci are used as antigens.

The reactivity of the antibody against each species of streptococci can be measured by ELISA method using as a test solution a standard strain suspended in a phosphate buffered saline (pH 7.4) (hereinafter, also referred to as "PBS"). Specific procedures include adding the PBS suspension of a standard strain at a certain concentration to each well of a 96-well immunoplate for adsorption, blocking, adding antibodies against *Streptococcus mutans* to the immunoplate, adding, for example, enzyme-labeled secondary antibodies, and measuring the enzyme activity. Thus, the reactivity of the antibody can be evaluated.

Preferably, each of the reactivities of this antibody against serotype c, e or f of *Streptococcus mutans* are equivalent to each other, in order to detect and quantitatively determine the total amount of *Streptococcus mutans* contained in a test solution. Here, the phrase "each of the reactivities against serotype c, e or f are equivalent to each other" means that, when antigen-antibody reaction is detected using a standard known immunoassay method wherein *Streptococcus mutans* of serotype c, e and f having equivalent amount of cells are used as antigens, reactions detected from these three serotypes are approximately equivalent to each other. Specifically, none of the reactions detected from the three serotypes is more than 2-fold, preferably more than 1.5-fold, greater than the others.

Furthermore, the reactivity of the antibody against *Streptococcus mutans* is not specifically limited. In view of measurement sensitivity, the cell concentration at the lower detection limit is 10⁶ cells/ml or less (5 x 10⁴ cells/well), preferably 2 x 10⁵ cells/ml or less (10⁴ cells/well), when 50 µl of each test solution containing *Streptococcus mutans* only is used as a sample, cells are immobilized in each well of a 96-well immunoplate, and then measurement is performed by direct ELISA method using antibodies of 10 ng to 500 ng/well.

### 2. Method for producing polyclonal antibody according to the present invention

The above-described polyclonal antibody according to the present invention can be preferably obtained by, for example, immunizing with the whole cells or the protease-treated cells of *Streptococcus mutans*, or the antigen extract of these cells so as to obtain polyclonal antibodies against *Streptococcus mutans*, and then, from the obtained polyclonal antibodies, collecting antibodies (hereinafter also referred to as "specific antibody") which react with *Streptococcus mutans.*

It is particularly preferable to produce polyclonal antibodies against *Streptococcus mutans* by immunizing using as antigens the protease-treated cells of *Streptococcus mutans* or the antigen extract thereof. Specifically, the method for producing a polyclonal antibody according to the present invention comprises the step of immunizing an immune animal with the whole cells of *Streptococcus mutans* treated with protease or the antigen extract thereof.

It is also preferred that, when specific antibodies are collected, the polyclonal antibody according to the present invention is produced by performing affinity purification using the protease-treated cells of *Streptococcus mutans* or the antigen extract thereof. Specifically, another method for producing the polyclonal antibody according to the present invention comprises a step of performing affinity purification using protease-treated whole cells of *Streptococcus mutans* or the antigen extract thereof.

A method for obtaining the antibody of the present invention is hereafter described in detail.

As an antigen for immunization, the whole cells of *Streptococcus mutans* of serotype c, e or f, or protease-treated cells of *Streptococcus mutans* of these 3 serotypes can be used. The antigen extract of the whole cells or the protease-treated cells can also be used. Each antigen (the whole cells or protease-treated cells, or the antigen extract thereof) derived from *Streptococcus mutans* of these serotypes may be used independently for immunizing an immune animal. Or the mixture of these antigens can also be used for immunization.

Examples of the whole cells that can be used herein include viable cells, dead cells treated, for example, with formalin or by heating, and frozen cells for storage.

Protease-treated cells can be prepared by treating whole cells with protease. As protease, various known proteases or the mixture thereof can be used without limitation. Examples of protease include pronase, proteinase K, pepsin, trypsin, chymotrypsin and the like.

Protease treatment can be performed, for example, by suspending whole cells in a buffer or the like prepared to have pH at around optimum pH for reaction of protease to be used therein, adding protease, and keeping the temperature at 15 to 50°C for 10 to 120 minutes, while mixing well. Specifically, after reaction terminates, for example, a reaction solution is centrifuged or filtered, so as to remove protease and collect protease-treated cells. To avoid contamination with protease, preferably, procedures for washing are performed 3 to 5 times. The washing procedures comprise suspending the above cells collected by centrifugation, filtration or the like in a buffer, such as PBS, and collecting the cells by the similar procedure.

Polysaccharide antigens can be extracted from the whole cells or protease-treated cells by a standard known method, such as a method which involves heating a cell suspension (Rants, L. A., Stanford. Med. Bull. 13: 290-291, 1955), or a method which involves extracting with nitrous acid (Tsutomu TAKEI, Journal of Osaka University Dental Society, 35:93-109, 1990). The extracted polysaccharide antigens can be used as antigens, or can also be used after purification.

These antigen extracts and the like can be used as antigens, or can be used in a form bound to carriers for immunization. As carriers, standard known carriers can be preferably used. Examples of such carriers include a keyhole limpet hemocyanin (KLH), bovine serum albumin (BSA), human serum albumin (HSA), chicken serum albumin, poly-L-lysine, polyalanyl lysine, dipalmityl lysine, tetanus toxoid, polysaccharide and the like.

Direct immunization with the whole cells or the protease-treated cells of *Streptococcus mutans* is particularly preferable for preparing the antibody of the present invention, because of the ease of preparing these antigens and the high antibody titer of the antibody prepared from these antigens.

As an animal to be immunized with the above antigen, an animal that can be generally used for obtaining an antibody can be used. Preferred examples include mammals, such as a mouse, goat, rabbit, guinea pig, or the like.

As a method for immunizing these animals with antigens, a standard known method can be employed without limitation. Specifically, an animal may be directly immunized with cells or cell extracts, or with a mixture of the cells or cell extracts with adjuvants added thereto. To directly immunize with cells, for example, suspension of cells which is obtained after culturing *Streptococcus mutans* in a brain heart infusion (hereinafter, abbreviated as "BHI") liquid medium or the like can be employed. Further, as an adjuvant, a standard known adjuvant can be preferably used. Examples of such an adjuvant include Freund's complete adjuvant, Freund's incomplete adjuvant, aluminum hydroxide adjuvant, pertussis adjuvant and the like.

After immunization of these animals with antigen, the blood is collected and the serum is prepared. Thus, a liquid containing polyclonal antibodies against *Streptococcus mutans* (hereinafter, the liquid may also be referred to as "antiserum") can be obtained.

The thus obtained antiserum can be used as the polyclonal antibody according to the present invention for use in an immunoassay, so far as the above-described M/S reactivity multiplication factor of the polyclonal antibodies contained in the antiserum is 1000 or more. However, antiserum normally contains proteins other than antibodies, such as albumin. Hence, to perform a specific assay with high sensitivity, it is preferable that a polyclonal antibody fraction is prepared by treating the obtained antiserum by a salting-out method, gel filtration method, ion exchange chromatography method, affinity chromatography method, electrophoresis or the like. For example, when a rabbit is immunized with the cells of *Streptococcus mutans* as antigen, antisera can be obtained after 4 weeks or later. The antisera are then treated by, for example, an affinity chromatography using protein A, so that a polyclonal antibody fraction (IgG fraction) can be obtained.

The above M/S reactivity multiplication factor of the thus obtained polyclonal antibody is measured. When the result is 1000 or more, it can be used for the assay of the present invention. When the M/S reactivity multiplication factor is less than 1000, polyclonal antibodies (specific antibody) which react with *Streptococcus mutans* are purified from the obtained polyclonal antibodies (hereinafter, also referred to as "antibody as raw material").

Generally, a polyclonal antibody is a mixture of various antibodies. Thus, some polyclonal antibodies obtained as described above also contain antibodies which react with other streptococci. This may cause a lower M/S reactivity multiplication factor of polyclonal antibodies. In this case, selective collection of specific antibodies from antibodies as raw material is repeated until the M/S reactivity multiplication factor becomes 1000 or more.

It is preferable to employ an affinity purification method to purify a specific antibody from antibodies as raw material. In the affinity purification method, specific antibodies are purified by allowing specific antibodies to be adsorbed to insoluble carriers (it may also be referred to as "antigen-immobilized carrier") to which at least one type selected from the group consisting of the protease-treated whole cells of *Streptococcus mutans,* the antigen extract thereof, the whole cells of *Streptococcus mutans* and the antigen extract thereof is immobilized, collecting the insoluble carriers, and then eluting specific antibodies from the insoluble carriers by a standard known method, for example by altering pH, ionic strength or permittivity.

Alternatively, at least one type selected from the group consisting of the protease-treated whole cells of *Streptococcus mutans*, the antigen extract thereof, the whole cells of *Streptococcus mutans* and the antigen extract thereof is used without using insoluble carriers, the selected cells or extracts are allowed to contact with antibodies as raw material, specific antibodies are adsorbed to separate, and then the specific antibodies may be eluted. In this case, there is a need to separate specific antibodies by absorption without using insoluble carriers. Therefore, the use of the protease-treated whole cells of *Streptococcus mutans* or the whole cells of *Streptococcus mutans* is preferred.

These methods prevent antibodies which have low reactivity or have no reactivity against *Streptococcus mutans* from being adsorbed to antigen-immobilized carriers, or the whole cells of *Streptococcus mutans* or the protease-treated cells. Therefore, the M/S reactivity multiplication factor of the adsorbed antibody becomes high.

In the method wherein affinity purification is performed using antigen-immobilized carriers, standard known carriers can be used as insoluble carriers without limitation. Examples of such a carrier include agarose, dextran, cellulose, polyacrylamide, polystyrene, chloroethylene, glass, silicone rubber, porosity silica beads and the like.

As a method for immobilizing the above antigens to these carriers, a chemical binding method, such as a cyanogen bromide activation method, and a method using physicochemical adsorption can be used without limitation.

A method directly using the whole cells or the protease-treated cells of *Streptococcus mutans* for affinity purification is more preferred, because the procedure is simple and the method allows simultaneous collection of a plurality of types of antibodies which react with a plurality of types of antigens present on the cell surface of *Streptococcus mutans.*

The whole cells or the protease-treated cells of *Streptococcus mutans* to be used in affinity purification can be prepared by a method similar to that employed for antigens for immunization.

As a method for contact and separation of antigen-immobilized carriers and antibodies as raw material in the affinity purification method, either a batch method or chromatography method can be used, or these methods can also be performed in combination.

The chromatography method is performed by charging a column with antigen-immobilized carriers, adding antibodies as raw material to the column, disposing of fractions which are not adsorbed and eluted from the column, and then collecting the adsorbed antibodies. The batch method is performed by mixing a liquid in which antigen-immobilized carriers are suspended with antibodies as raw material, allowing antigen-antibody reaction to sufficiently proceed, collecting precipitate by, for example, centrifugation, and then eluting antibodies from the precipitate.

The method for contacting free whole cells or protease-treated cells of *Streptococcus mutans* with antibodies as raw materials and the method of separating these cells from antibodies as raw materials can be performed in the manner similar to the above batch method. Specifically, the method can be performed by mixing a cell suspension with antibodies as raw material, allowing antigen-antibody reaction to proceed sufficiently, and then collecting precipitate by, for example, centrifugation.

Examples of a preferred combination of an antigen to be used for immunization and a technique for affinity purification so that the M/S reactivity multiplication factor is 1000 or more, include:
a) a combination wherein immunization is performed using the protease-treated cells of *Streptococcus mutans* or the antigen extract thereof, and then affinity purification is performed using the whole cells or the protease-treated cells of *Streptococcus mutans*, or the antigen extract of these cells, and
b) a combination wherein immunization is performed using the whole cells of *Streptococcus mutans* or the antigen extract thereof, and then affinity purification is performed using the protease-treated cells or the antigen extract thereof.

### 3. Method for measuring Streptococcus mutans according to the present invention

The use of the above polyclonal antibody according to the present invention makes it possible to specifically measure or to detect *Streptococcus mutans* contained in a test solution with high sensitivity. Specifically, even when other species of streptococci are present in a test solution, *Streptococcus mutans* at a concentration of about 10⁵ cells/ml can be detected using the above polyclonal antibody according to the present invention.

Particularly, in this measurement method, various polyclonal antibodies obtained as described above can be used independently, and can also be used as a mixture of polyclonal antibodies having different properties. For example, the reactivities of polyclonal antibodies against *Streptococcus mutans* of serotype c, e and f can be made equivalent to each other for use in this method by appropriately mixing a polyclonal antibody having reactivity against *Streptococcus mutans* of serotype c which is twice or more greater than its reactivity against serotype e or f, a polyclonal antibody having reactivity against serotype e which is twice or more greater than its reactivity against serotype c or f, and a polyclonal antibody having reactivity against serotype f which is twice or more greater than its reactivity against serotype c or e.

A test solution to be used in this method is not specifically limited, so far as it contains *Streptococcus mutans*, such as an original test solution collected from a subject, the treated original test solution or the like. Examples of such an original test solution that can be used herein include saliva, dental plaque, cell culture solution, suspension of cultured cells and the like. As a sample for checking dental caries risk, test solution containing saliva and dental plaque are preferably used. In addition, saliva and dental plaque may be independently contained or contained as a mixture thereof in an original test solution.

For example, an original test solution containing only dental plaque may be prepared using dental plaque which is collected after washing oral cavity by gargling to remove a saliva component. Dental plaque can be collected by a standard known method, such as a toothpick, swab or spatula from an oral certain site, and can also be collected randomly from oral cavity. The thus collected dental plaque is suspended in a liquid, such as a buffer, thereby preparing an original test solution. For example, when a mixture of dental plaque and saliva is used as an original test solution, it can be collected by asking a subject to chew a masticatory substance, such as a paraffin pellet, for 30 seconds to 10 minutes, and then to spit out secreted saliva.

Further, an original test solution containing only saliva can be prepared by using saliva which is collected by a standard known tool, such as a syringe or pipette. The thus collected saliva may be used or used after appropriate dilution with a buffer, as an original test solution. These original test solutions can be used as test solutions in measurement of *Streptococcus mutans*. However, preferably a test solution is prepared by a standard known treatment for dispersing insoluble substances contained in an original test solution, ultrasonication or mixing and stirring with a crushing agent, such as glass beads, and then subjected to analysis.

In addition, the measurement of *Streptococcus mutans* can also be performed by measuring the antigen extract in the test solution which is prepared by performing extraction treatment on *Streptococcus mutans* in the above original test solution, because the quantity of the antigen extract correlates with the quantity of *Streptococcus mutans.* As a method for extracting antigen, a known method can be used without limitation. Examples of such a method include a method which involves heat treatment (Rants, L. A., Stanford. Med. Bull. 13: 290-291, 1955), an extraction method which uses nitrous acid, alkali, salt, a chelating agent, chaotropic ion or a surfactant (Tsutomu TAKEI, Journal of Osaka University Dental Society, 35: 93-109, 1990), and a method which involves treating with enzyme which disrupts bacterial cell membrane structure (JP Patent Publication (Unexamined Application) No. 9-178752). The methods which use a liquid containing antigen extract as a test solution are particularly preferred, because the antigen extract can be detected with higher sensitivity.

In the measurement method of the present invention, the amount or concentration of *Streptococcus mutans* is determined by measuring a complex which is formed by antigen-antibody reaction between *Streptococcus mutans* or the antigen extract obtained from *Streptococcus mutans* and the polyclonal antibody of the present invention. At this time, the concentration of *Streptococcus mutans* can be obtained from the above measured values of the complexes based on the relation between the measured value of the complexes and the concentration of *Streptococcus mutans* determined by performing measurement for some standard test solutions that have been prepared, for example, using standard strains so as to have known concentrations of *Streptococcus mutans.* The thus determined concentration of *Streptococcus mutans* in an original test solution is consistent with the concentration of *Streptococcus mutans* (concentration unit: cells/ml) in an original test solution measured by counting *Streptococcus mutans* according to a standard known bacteriological technique, such as cultivation.

Moreover, concentration of *Streptococcus mutans* (concentration unit: cells/ml) in an original test solution can be measured by counting *Streptococcus mutans* according to a standard known bacteriological technique, such as cultivation. For example, when concentration is measured by a culture method, concentration of *Streptococcus mutans* (concentration unit: cells/ml) in an original test solution can be measured by performing treatment, such as ultrasonication, to distribute cells, on a suspension prepared by appropriate dilution of an original test solution, pipetting the suspension on a BHI medium plate, counting the number of colonies formed on the plate, and then multiplying by the dilution ratio of the original test solution.

However, an original test solution contains *Streptococcus sobrinus* or other oral bacteria in addition to *Streptococcus mutans.* Hence, colonies formed on a medium plate should be correctly classified into colonies of *Streptococcus mutans* and those of other bacteria, so as to be able to correctly count the number of *Streptococcus mutans* only. Colonies formed on a medium plate can be classified by, for example, a biochemical technique, such as a sugar fermentation method, an immunological method using specific antibodies, or a genetic technique using DNA probes.

In this measurement method, *Streptococcus mutans* in the above-described test solution is measured by an immunoassay using the above polyclonal antibody. The immunoassay is capable of rapidly and conveniently measuring antigen in a test solution containing contaminants.

An immunoassay is not specifically limited, and a standard known immunoassay can be used without limitation. Examples of such an immunoassay include those known as a method for measuring antigen by antigen-antibody reaction, such as an immune agglutination method, an optical immunoassay, a labeled immunoassay, or a combination thereof. The form of a measurement reagent for performing these immunoassays is not specifically limited, so far as it is a reagent for an immunoassay containing the antibody of the present invention. The reagent that can be employed herein may be in a variety of forms including a solution containing the antibody of the present invention; insoluble carriers, such as a particle, membrane or the like, to which the antibody of the present invention is immobilized, or the suspension thereof; the antibody of the present invention to which a labeling substance, such as a radioactive substance, enzyme, various coloring agents, colloid substances, or various coloration particles, is bound, or the solution or suspension thereof; or an insoluble carrier, such as a particle or membrane, to which the thus labeled antibody of the present invention is immobilized, or the suspension thereof; and combinations of these substances, and the like.

Measurement methods using these reagents for immunoassays are described below.

### [Immune agglutination method]

This method is for detecting and quantitatively determining *Streptococcus mutans* contained in a test solution using agglutination reaction of insoluble carriers based on antigen-antibody reaction. Examples of a semi-quantitative method include a latex agglutination method, a microtiter method and the like. Examples of a quantitative method include a latex quantitative method and the like.

For example, when the latex agglutination method is used for immunologically measuring *Streptococcus mutans* in a test solution, *Streptococcus mutans* can be measured by preparing a measurement reagent containing particles which are sensitized with antibodies by immobilizing the polyclonal antibodies according to the present invention to latex beads, mixing the measurement reagent with a test solution, and then detecting the degree of agglutination of the sensitized particles after antigen-antibody reaction by visual inspection, an optical measurement method or the like.

### [Optical immunoassay]

This method means a method for optically detecting the presence or absence of antigen-antibody reaction, when antigen-antibody reaction is performed by allowing the polyclonal antibody according to the present invention to contact with a test solution. Examples of such a method include a method which involves detecting a change in turbidity of agglutinates resulting from antigen-antibody reaction; a method which involves detecting a change in transmittance provided by allowing a test solution to contact with a measurement reagent, wherein the polyclonal antibodies according to the present invention are immobilized on a transparent support, and then allowing particulate cells to bind onto the transparent support as a result of antigen-antibody reaction; and a method which involves detecting, a change in refractive index, as a change in transmitted beam, surface plasmon wave or the like, when a test solution is allowed to contact with a thin layer (hereinafter, also referred to as an antibody layer) to which the polyclonal antibodies according to the present invention are immobilized to cause the change in refractive index of the antibody layer formed as a result of antigen-antibody reaction.

### [Labeled immunoassay]

This method is for detecting and quantitatively determining *Streptococcus mutans* in a test solution by allowing a test solution to contact with a measurement reagent containing labeled antibodies which are prepared by binding various labeling substances, such as a radioactive substance, enzyme, various pigments, colloidal substances or various particles to the polyclonal antibodies according to the present invention to perform antigen-antibody reaction; and then detecting labeled antibodies bound to antigens in the test solution. Specifically, the method is for detecting and quantitatively determining *Streptococcus mutans* in a test solution by measuring radioactivity, enzyme activity, fluorescence intensity, coloration or the like originating from labeling substances.

*Streptococcus mutans* in a test solution can be detected and quantitatively determined by another example of this method which involves the steps of allowing the test solution to contact with a measurement reagent containing insoluble carriers (for example, particles, membrane, ELISA plates or the like) to which the polyclonal antibodies according to the present invention are immobilized to perform antigen-antibody reaction; allowing insoluble carriers to contact with another measurement reagent containing the polyclonal antibodies (according to the present invention) labeled with labeling substances to perform another antigen-antibody reaction; and then measuring the amount of labeled substances bound to the polyclonal antibodies according to the present invention. *Streptococcus mutans* in a test solution can also be detected and quantitatively determined by still another example of this method which involves mixing a test solution with the cells of *Streptococcus mutans* or the bacterial surface antigens labeled with labeling substances, allowing this mixture to contact with a measurement reagent containing insoluble carriers to which the polyclonal antibodies according to the present invention are immobilized to perform antigen-antibody reaction; and then measuring the amount of labeled substances bound to the polyclonal antibodies according to the present invention.

Examples of a labeling substance that can be used herein include a radioactive substance, such as radioactive iodine or radioactive carbon; an enzyme, such as peroxidase, alkaline phosphatase or galactosidase; various pigments, such as fluorescent dyes, e.g., fluorescein isothiocyanate and tetramethylrhodamine; a colloid, such as colloidal gold or colloidal carbon; and various particles, such as a coloration latex particle.

When an enzyme is labeled, direct labeling by covalent binding of a thiol group with a maleimide group, an amino group with an aldehyde group, or the like, is performed. Alternatively, a method which involves labeling via a biotin-avidin complex can also be employed. Further, when alkaline phosphatase is used as an enzyme to be labeled, a chemiluminescence substance, such as a dioxetane derivative, or when peroxidase is used, a chemiluminescence substance, such as a luminol derivative may be used as a substrate for enzyme, thereby detecting luminescence from the substrate.

To these various labeled immunoassays, generally employed manipulations, procedures or the like can be appropriately applied. These methods can be performed according to a known non-competitive or competitive method, a sandwich method or the like. In addition, substances which can be bound to the polyclonal antibody according to the present invention, such as a secondary antibody or protein A which is labeled with any one of the above labeling substances, can be used together with the polyclonal antibody according to the present invention for detection and quantitative determination *of Streptococcus mutans.*

Moreover, a combination of a plurality of antibodies can also be used for detection and quantitative determination of *Streptococcus mutans*, if necessary. For example, in immunoassay based on the principle of the sandwich method, a monoclonal antibody against *Streptococcus mutans* can be used as an antibody to be immobilized to a solid phase, and the polyclonal antibody according to the present invention can be used as a labeled antibody.

In the above labeled immunoassay, conventionally employed methods can be used according to a label to be used, without specific limitation. In particular, when highly sensitive quantitative measurement is required, examples of methods including a radioimmunoassay which uses a radioactive substance as a label, an enzyme immunoassay which uses enzyme as a label, a fluorescent immunoassay which uses a fluorescent dye as a label, and a chemiluminescent immunoassay which uses a chemiluminescent substance (a substrate for an enzyme) as a label are preferably employed because of their high quantitativity. In addition, methods which use colloidal substances or various other particles as labels, such as a flow-through immunoassay, an immunochromatography method or a latex agglutination method, are characterized by their simple procedures.

Among these various immunoassays, the latex assay system and the enzyme immunoassay are preferred as a testing method employed for group medical examination or the like, because a large number of test solutions can be treated by these methods using an autoanalyzer. Further, the flow-through immunoassay, immunochromatography and latex agglutination method enable rapid measurement without the need of special knowledge or a device, in addition to involving simple procedures. Thus, these methods are also preferred as a general testing method, since they can also be performed at a dental office or home.

An immunochromatography method can be performed at a dental office or home, and we have found that *Streptococcus mutans* in a test solution can be measured with high sensitivity using the antibody of the present invention in a measurement reagent which is referred to as an immunochromatography test strip (also referred to as simply a "strip") that is generally used in the immunochromatography method, to enhance the detection limit. Accordingly, an immunochromatography method using a strip utilizing the above polyclonal antibody according to the present invention (hereinafter, also referred to as "the immunochromatography method according to the present invention") can be a superior general testing method for determining the risk of dental caries. This method will be described in detail below by referring to the drawings.

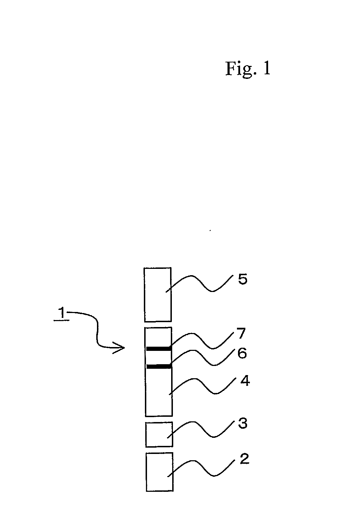

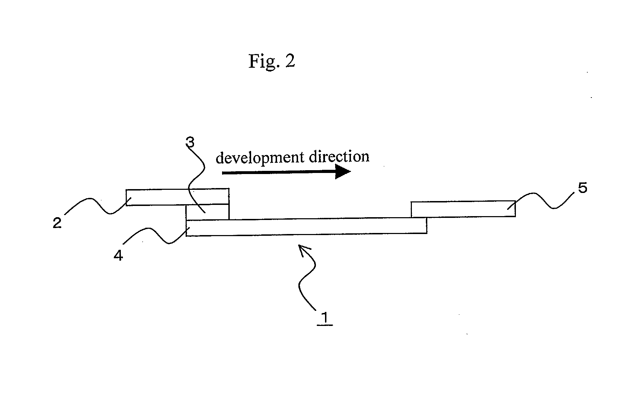

Figures 1 and 2 show the structure of a typical strip 1 that can be preferably used in the immunochromatography method according to the present invention. The strip 1 has a structure similar to a standard strip, wherein each member comprises a purpose-oriented porous support. Specifically, the strip 1 has a structure wherein a sample pad 2 for temporarily absorbing and retaining a test solution, a conjugate pad 3 for temporarily retaining a labeled antibody, a development membrane 4 to which an antibody for detection is immobilized, and an absorbent pad 5 for absorbing the test solution which flows from the sample pad 2 are coupled in this order.

The conjugate pad 3 in the strip 1 retains an antibody (hereinafter, referred to as "labeled antibody") coated and dried thereon for labeling *Streptococcus mutans* in a test solution. As the labeled antibody, for example, an antibody which is labeled with a labeling substance, such as colloidal gold, is used. On a detection line 6 located on the above development membrane 4, an antibody for detection for capturing *Streptococcus mutans* in a test solution is immobilized. The polyclonal antibody according to the present invention may be used as both the above labeled antibody and the above antibody for detection, or the polyclonal antibody according to the present invention and a monoclonal antibody against *Streptococcus mutans* may be used in combination. Specifically, in the strip 1, the polyclonal antibody according to the present invention is used as at least one of the above labeled antibody and the antibody for detection.

A material to be used for the porous support which composes each of the above members is not specifically limited, and is appropriately selected from a hygroscopic material, a porous material, a fiber material and the like, according to the purposes of each member. For example, preferably, filter paper, blotting paper or the like is used as the sample pad 2, and fibrous glass fabric, polypropylene nonwoven fabric, glass filter or the like is used as the conjugate pad 3. In addition, preferably, nitrocellulose, nitrocellulose containing cellulose acetate or the like is used as the development membrane 4, and filter paper, blotting paper or the like is used as the absorbent pad 5.

In addition, a method for immobilizing antibodies for detection and control antibodies to the development membrane 4 is not specifically limited. A standard known physical adsorption method and covalent binding method can be used without limitation. The mixture of antibodies and other proteins or the like also can be immobilized. Furthermore, in order to inhibit non-specific adsorption of the component of a test solution to the development membrane 4, or to improve wettability of a test solution to the development membrane 4, the development membrane 4 can be subjected to blocking treatment by a known method using a protein, a lipid, a high molecular compound or the like, after immobilization of antibodies. The protein or the like which is used for the blocking treatment is not specifically limited, and preferably, BSA, skim milk or the like that is used in a general immunoassay for inhibiting non-specific reaction is used. In addition, for a test solution to uniformly develop in the development membrane 4, the water-absorbing property of the development membrane 4 can be adjusted by allowing a hydrophilic polymer or surfactant to impregnate the surface of the development membrane 4, or coating a hydrophilic polymer or surfactant thereon.

In addition, the conjugate pad 3 is preferably prepared by blocking with a water-soluble polymer or a sugar, such as saccharose, adding labeled antibodies after blocking, and then drying; or by mixing labeled antibodies with water-soluble polymers or a sugar, such as saccharose, coating the mixture on the conjugate pad 3, and then drying, so that complexes of *Streptococcus mutans* and the labeled antibodies are easily released from the site when a test solution is added.

As the above water-soluble polymer, for example, polyvinylpyrrolidone, polyvinyl alcohol, polyethylene glycol, cellulose ether (methyl cellulose, ethyl cellulose, carboxymethyl-cellulose, carboxyethyl cellulose, oxyethyl cellulose, cyanoethyl cellulose and the like), gelatine and the like can be used.

As a labeling substance to prepare a labeled antibody, labeling substances which can be visually observed, such as colloidal gold, colloidal carbon or coloring latex can be used. Alternatively, a radioactive substance or a fluorescent substance can also be used. When an enzyme is used as a labeling substance, a substrate is added after antigen-antibody reaction, and then compounds formed by the enzyme reaction can also be detected. A method for labeling the polyclonal antibody according to the present invention is not specifically limited, and labeling can be performed by a generally employed standard method.

The immunochromatography method according to the present invention enables measurement of the presence or absence or amount of *Streptococcus mutans* in a test solution, by the steps of adding a test solution to the sample pad 2 located in the neighborhood of the conjugate pad 3 of the above strip 1; allowing the strip 1 to stand at room temperature for 1 to 30 minutes in order to allow labeled antibodies to develop together with the test solution to the detection line 6; based on the principle of the Sandwich method, capturing *Streptococcus* *mutans* bound to the labeled antibodies on the detection line 6 using antibodies for detection; and detecting the labeled antibodies bound to the captured *Streptococcus mutans.* At this time, development of labeled antibodies with a test solution can be confirmed by detecting the labeled antibodies at a position where antibodies reacting with the labeled antibodies have been immobilized on a control determination line 7 located downstream of the above detection line 6.

The position, order, shape, number or the like of the detection line 6 or the control determination line 7 on the development membrane 4 as shown in Fig. 1 is an example, and is not specifically limited.

The immunochromatography method using the strip 1 wherein labeled antibodies are coated and dried is as described above. Measurement can also be performed by mixing labeled antibodies with a test solution for antigen-antibody reaction to proceed, and then adding the mixed solution to one end of a porous support to which antibodies for detection are immobilized.

The immunochromatography method is performed by, for example, the following procedures, so that the amount of *Streptococcus mutans* present in the oral cavity of a subject can be rapidly and conveniently found and the risk of dental caries can be diagnosed and determined.

Specifically, (1) dental plaque or saliva is collected as an original test solution from oral cavity (dental plaque is collected using a spatula or a cotton swab, or saliva is collected by asking a subject to chew paraffin gum), (2) when an original test solution is saliva, it is used as test solution without treatment, or a test solution is prepared by suspending saliva in a diluting solution, or when an original test solution is dental plaque, it is suspended in a diluting solution, and then subjected to a treatment for dispersing dental plaque, such as ultrasonication, if necessary, or extraction treatment is performed on the original test solution to prepare a test solution, (3) a test solution (for example, 100 to 200 µl) is sampled, and added to the sample pad of the above strip, and then allowed to stand for a certain time (for example, 1 to 30 minutes), so that the presence or absence of *Streptococcus mutans* or the amount thereof can be found by determining the presence or absence or the state of coloration of the detection line 6 and the control determination line 7 on the strip.

For example, when colloidal gold is used as a labeling substance, the degree of coloration by colloidal gold on the detection line 6 changes as shown in Table 1 according to the concentration of *Streptococcus mutans* in a test solution. Thus, the risk of dental caries can be diagnosed and determined according to the criteria of Table 2.

**[Table 1]**

| | | | | |
|---|---|---|---|---|
| Cell count of *Streptococcus mutans* (cells/ml) | 10⁷ | 10⁶ | 10⁵ | <10⁵ |
| Color of determination line | dark red | Red | light red | colorless |
| Determination | +++ | ++ | + | - |

**[Table 2]**

| Determination line | Control line | Determination | Risk of dental caries |
|---|---|---|---|
| Dark red | red to purple | positive | high risk |
| Red | red to purple | positive | high risk |
| Light red | red to purple | positive | moderate risk |
| Colorless | red to purple | negative | low risk |
| Red to purple | colorless | re-examination | - |
| Colorless | colorless | re-examination | - |

Use of the immunochromatography method according to the present invention in this way enables the amount of *Streptococcus mutans*, which is particularly important as a causative bacterium of dental caries, to be measured rapidly and conveniently, so that the risk of dental caries of a subject can be diagnosed and determined.

### EXAMPLES

The present invention is more specifically described by the following Examples. However, these examples are not intended to limit the scope of the present invention.

### Example 1 [Preparation of polyclonal antibody, and evaluation of reactivity of antibody]

### (1) [Preparation of cell suspension]

3.7 g of BHI (DIFCO) was dissolved in 100 ml of extra pure water, and then the solution was autoclaved, thereby preparing a BHI liquid medium. Ingbritt *(Streptococcus mutans*, serotype c) was cultured in 2 ml of BHI liquid medium at 37°C for 5 hours under anaerobic conditions (N₂:H₂:CO₂=80:10:10). The culture solution was centrifuged at 4000 x g for 5 minutes to remove medium components in the supernatant, so that cell precipitate was collected.

Next, the precipitate was suspended in 5 ml of PBS, and then a similar centrifugation procedure was performed thrice so as to wash the precipitate. The resulting cell precipitate was suspended in PBS to adjust at A₆₀₀=1.0, thereby preparing a cell sample suspension. In addition, when the cell sample suspension was subjected to ultrasonication, appropriately diluted, and added onto BHI medium plate, and the number of colonies formed on the plate counted and the cell count then multiplied by the dilution ratio of the cell suspension, the cell concentration of the cell suspension was about 1 x 10⁹ cells/ml.

In the manner similar to the above procedures, cell suspensions of P4 *(Streptococcus mutans*, serotype e), OMZ175 *(Streptococcus mutans*, serotype f), Challis *(Streptococcus gordonii*), IFO13956 (*Streptococcus salivarius*), ATCC35037 *(Streptococcus oralis*), ATCC15911 *(Streptococcus mitis),* ATCC10556 *(Streptococcus sanguis*) were prepared. Here, Ingbritt, P4, OMZ175, Challis and the like represent the names of standard strains; and ATCC35037, IFO13956 and the like represent Accession Nos. of standard strains.

### (2) [Preparation of antiserum against Streptococcus mutans]

Ingbritt was cultured in 100 ml of autoclaved BHI liquid medium at 37°C for 5 hours under anaerobic conditions. The culture solution was centrifuged at 4000 x g for 5 minutes to remove medium components in the supernatant, and then cell precipitate was collected. Next, the precipitate was suspended in 100 ml of PBS, and then a similar centrifugation procedure was performed thrice to wash the precipitate. The cell precipitate was suspended in PBS to adjust at A₆₀₀=1.0, thereby preparing an Ingbritt cell suspension.

Alternatively, after Ingbritt cells cultured in BHI medium were washed, the cells were suspended in 0.1 M Tris hydrochloride buffer (pH 8.0), and then the suspension was adjusted at A₆₀₀=15. To the suspension, pronase (Wako Pure Chemical Industries Ltd.) was added at 5 mg/ml, and then the solution was kept at 37°C for 1 hour. After reaction, cell precipitate was collected by centrifugation. Subsequently, the precipitate was suspended in 10 ml of PBS, and then similar centrifugation procedures were performed thrice to wash the precipitate. The cell precipitate was suspended in PBS to adjust at A₆₀₀=1.0, thereby preparing a protease-treated Ingbritt cell suspension.

Immunization was performed as follows.

During the 1^{st} week, 0.5 ml of the cell suspension or the protease-treated cell suspension was injected to the auricular vein of a rabbit for 5 days in a row (5 times). During the 2^{nd} week, 1.0 ml of the cell suspension or the protease-treated cell suspension was injected to the auricular vein of the rabbit for 5 days in a row (5 times). During the 3^{rd} week, 2.0 ml of the cell suspension or protease-treated cell suspension was injected to the auricular vein of the rabbit for 5 days in a row (5 times). During the 4^{th} week, immunization was performed in the manner similar to the 3^{rd} week. Elevated titer levels were confirmed based on the degree of agglutination reaction of cells using a slide glass. Then, 1 week after the final immunization, blood was collected according to a standard technique, so that antiserum against *Streptococcus mutans* was obtained.

### (3) [Purification of polyclonal antibody against Streptococcus mutans]

According to the method of (2), Ingbritt or protease-treated Ingbritt cultured in 1 L of BHI liquid medium was washed thrice with PBS, washed thrice with 0.1 M glycine hydrochloride buffer (pH 2.0), and then washed thrice with PBS, thereby preparing a cell suspension and a protease-treated cell suspension (A₆₀₀=12.5).

Next, the protease-treated cell suspension and 0.5 ml of the antiserum obtained by immunizing with the protease-treated cell suspension prepared in (2) were mixed, or the cell suspension and 0.5 ml of the antiserum obtained by immunizing with the protease-treated cell suspension prepared in (2) were mixed; or the protease-treated cell suspension and 0.5 ml of the antiserum obtained by immunizing with the cell suspension prepared in (2) were mixed. Then, the mixed solution was allowed to react at 4°C for 60 minutes. The mixed solution was centrifuged at 4000 x g for 5 minutes to collect each type of the cells. Each type of the cells was suspended in 10 ml of PBS, and then similar centrifugation procedures were repeated thrice for washing.

Subsequently, each type of the cells was suspended in 0.5 ml of 0.1 M glycine hydrochloride buffer (pH 2.0), adsorbed antibodies were eluted, and then supernatant was collected by centrifugation. 1 M Tris-hydrochloride (pH 9.0) was added to the supernatant to adjust at pH 7.4. Similar elution procedures were performed 4 times, and then the protein quantity of each fraction was measured using absorbance at 280 nm.

Next, the above eluate was added to a column charged with 1 ml of Protein A-Sepharose (Amersham Pharmacia Biotech) which had been previously equilibrated with PBS. The column was washed with 5 ml of PBS, followed by elution with 5 ml of 0.1 M glycine-hydrochloride buffer (pH 3.0). Immediately after elution, 1 M Tris-hydrochloride (pH 9.0) was added to adjust at pH 7.4. Elution fraction of IgG was confirmed by measuring A₂₈₀.

Thus, 0.2 mg of polyclonal antibody "M-1" was obtained by purifying, using the protease-treated cells, the antiserum (0.5 ml) obtained using the protease-treated cell suspension as antigen. Further, 1 mg of polyclonal antibody "M-2" was obtained by purifying, using the cells, the antiserum (0.5 ml) obtained using the protease-treated cell suspension as antigen. Furthermore, 1 mg of polyclonal antibody "M-3" was obtained by purifying, using the protease-treated cells, the antiserum (0.5 ml) obtained using the cell suspension as antigen.

### (4) [Evaluation by direct ELISA method of polyclonal antibody against Streptococcus mutans]

Reactivities of the polyclonal antibodies (M-1, M-2 and M-3) against *Streptococcus mutans* purified in (3) above against various strains were evaluated by the following direct ELISA method.

Specifically, the cell suspension obtained by the method of (1) above was diluted at 1 x 10⁴ to 10⁹ cells/ml using 0.1 M carbonate buffer (pH 9.0). 50 µl of the diluted solution was added to each well of a 96-well immunoplate (MaxiSorp, manufactured by NUNC), and then allowed to stand at 4°C for 12 hours for immobilization. Then, the solution was removed from the immunoplate, and then each well was washed thrice with 300 µl of PBS.

Next, 300 µl of 2% BSA-0.1 M carbonate buffer (pH 9.0) was added to each well of the immunoplate, and then allowed to stand at 37°C for 2 hours. Then, 2% BSA-carbonate buffer (pH 9.0) was removed from the immunoplate, and the wells were then washed thrice with 300 µl of 0.05% Tween 20-PBS (pH7.4).

Subsequently, M-1, M-2 or M-3 was diluted at 1.0 µg/ml in 1% BSA-0.05 % Tween 20-PBS (pH 7.4). 50 µl of the diluted solution was added to each well of the immunoplate, and then allowed to stand at 37°C for 1 hour. After the solution was removed from the immunoplate, the immunoplate was washed thrice with 300 µl of 0.05% Tween 20-PBS (pH 7.4).

Next, alkaline phosphatase-labeled anti-rabbit IgG (Fc) polyclonal antibody (goat) (Cappel) was diluted at 10 µg/ml in 1% BSA-0.05 % Tween 20-PBS (pH 7.4). 50 µl of the diluted solution was added to each well of the immunoplate, and then allowed to stand at 37°C for 1 hour. After the solution was removed from the immunoplate, the immunoplate was washed thrice with 300 µl of 0.05% Tween 20-PBS (pH 7.4).

Subsequently, as a chromogenic substrate solution, 50 µl of a 2-ethanolamine aqueous solution of p-nitrophenyl phosphate (BIORAD) was added to each well for reaction to proceed at room temperature for 20 minutes. After reaction, 50 µl of 0.4 M NaOH was added to each well to stop reaction. Then, the absorbance at 405 nm was measured. Table 3 shows the result for M-1, Table 4 shows the result for M-2, and Table 5 shows the result for M-3.

**[Table 3]**

| Strain | Species/Serotype | Cell count (cells/well) | A₄₀₅ |
|---|---|---|---|
| Ingbritt | *Streptococcus mutans*/c | 5×10² | 0.003 |
| Ingbritt | *Streptococcus mutans*/c | 5×10³ | 0.010 |
| Ingbritt | *Streptococcus mutans*/c | 5×10⁴ | 0.060 |
| Ingbritt | *Streptococcus mutans*/c | 5×10⁵ | 0.553 |
| Ingbritt | *Streptococcus mutans*/c | 5×10⁶ | 1.544 |
| Ingbritt | *Streptococcus mutans*/c | 5×10⁷ | >2.000 |
| P4 | *Streptococcus mutans*/e | 5×10² | 0.004 |
| P4 | *Streptococcus mutans*/e | 5×10³ | 0.010 |
| P4 | *Streptococcus mutans*/e | 5×10⁴ | 0.050 |
| P4 | *Streptococcus mutans*/e | 5×10⁵ | 0.487 |
| P4 | *Streptococcus mutans*/e | 5×10⁶ | 1.323 |
| P4 | *Streptococcus mutans*/e | 5×10⁷ | >2.000 |
| OMZ175 | *Streptococcus mutans*/f | 5×10² | 0.003 |
| OMZ175 | *Streptococcus mutans*/f | 5×10³ | 0.012 |
| OMZ175 | *Streptococcus mutans*/f | 5×10⁴ | 0.040 |
| OMZ175 | *Streptococcus mutans*/f | 5×10⁵ | 0.303 |
| OMZ175 | *Streptococcus mutans*/f | 5×10⁶ | 1.032 |
| OMZ175 | *Streptococcus mutans*/f | 5×10⁷ | 1.877 |
| Challis | *Streptococcus gordonii* | 5×10⁵ | 0.002 |
| Challis | *Streptococcus gordonii* | 5×10⁶ | 0.005 |
| Challis | *Streptococcus gordonii* | 5×10⁷ | 0.011 |
| IFO13956 | *Streptococcus salivarius* | 5×10⁵ | 0.005 |
| IFO13956 | *Streptococcus salivarius* | 5×10⁶ | 0.004 |
| IFO13956 | *Streptococcus salivarius* | 5×10⁷ | 0.008 |
| ATCC35037 | *Streptococcus oralis* | 5×10⁵ | 0.004 |
| ATCC35037 | *Streptococcus oralis* | 5×10⁶ | 0.003 |
| ATCC35037 | *Streptococcus oralis* | 5×10⁷ | 0.005 |
| ATCC15911 | *Streptococcus mitis* | 5×10⁵ | 0.003 |
| ATCC15911 | *Streptococcus mitis* | 5×10⁶ | 0.004 |
| ATCC15911 | *Streptococcus mitis* | 5×10⁷ | 0.007 |
| ATCC10556 | *Streptococcus sanguis* | 5×10⁵ | 0.004 |
| ATCC10556 | *Streptococcus sanguis* | 5×10⁶ | 0.003 |
| ATCC10556 | *Streptococcus sanguis* | 5×10⁷ | 0.010 |

**[Table 4]**

| Strain | Species/Serotype | Cell count (cells/well) | A₄₀₅ |
|---|---|---|---|
| Ingbritt | *Streptococcus mutans*/c | 5×10² | 0.003 |
| Ingbritt | *Streptococcus mutans*/c | 5×10³ | 0.014 |
| Ingbritt | *Streptococcus mutans*/c | 5×10⁴ | 0.071 |
| Ingbritt | *Streptococcus mutans*/c | 5×10⁵ | 0.581 |
| Ingbritt | *Streptococcus mutans*/c | 5×10⁶ | 1.501 |
| Ingbritt | *Streptococcus mutans*/c | 5×10⁷ | >2.000 |
| P4 | *Streptococcus mutans*/e | 5×10² | 0.003 |
| P4 | *Streptococcus mutans*/e | 5×10³ | 0.011 |
| P4 | *Streptococcus mutans*/e | 5×10⁴ | 0.056 |
| P4 | *Streptococcus mutans*/e | 5×10⁵ | 0.423 |
| P4 | *Streptococcus mutans*/e | 5×10⁶ | 1.356 |
| P4 | *Streptococcus mutans*/e | 5×10⁷ | >2.000 |
| OMZ175 | *Streptococcus mutans*/f | 5×10² | 0.005 |
| OMZ175 | *Streptococcus mutans*/f | 5×10³ | 0.011 |
| OMZ175 | *Streptococcus mutans*/f | 5×10⁴ | 0.044 |
| OMZ175 | *Streptococcus mutans*/f | 5×10⁵ | 0.312 |
| OMZ175 | *Streptococcus mutans*/f | 5×10⁶ | 1.045 |
| OMZ175 | *Streptococcus mutans*/f | 5×10⁷ | 1.852 |
| Challis | *Streptococcus gordonii* | 5×10⁵ | 0.003 |
| Challis | *Streptococcus gordonii* | 5×10⁶ | 0.004 |
| Challis | *Streptococcus gordonii* | 5×10⁷ | 0.010 |
| IFO13956 | *Streptococcus salivarius* | 5×10⁵ | 0.005 |
| IFO13956 | *Streptococcus salivarius* | 5×10⁶ | 0.007 |
| IFO13956 | *Streptococcus salivarius* | 5×10⁷ | 0.009 |
| ATCC35037 | *Streptococcus oralis* | 5×10⁵ | 0.003 |
| ATCC35037 | *Streptococcus oralis* | 5×10⁶ | 0.005 |
| ATCC35037 | *Streptococcus oralis* | 5×10⁷ | 0.00⁶ |
| ATCC15911 | *Streptococcus mitis* | 5×10⁵ | 0.002 |
| ATCC15911 | *Streptococcus mitis* | 5×10⁶ | 0.003 |
| ATCC15911 | *Streptococcus mitis* | 5×10⁷ | 0.008 |
| ATCC10556 | *Streptococcus sanguis* | 5×10⁵ | 0.003 |
| ATCC10556 | *Streptococcus sanguis* | 5×10⁶ | 0.005 |
| ATCC10556 | *Streptococcus sanguis* | 5×10⁷ | 0.012 |

**[Table 5]**

| Strain | Species/Serotype | Cell count (cells/well) | A₄₀₅ |
|---|---|---|---|
| Ingbritt | *Streptococcus mutans*/c | 5 × 10² | 0.004 |
| Ingbritt | *Streptococcus mutans*/c | 5 × 10³ | 0.010 |
| Ingbritt | *Streptococcus mutans*/c | 5 × 10⁴ | 0.051 |
| Ingbritt | *Streptococcus mutans*/c | 5 × 10⁵ | 0.566 |
| Ingbritt | *Streptococcus mutans*/c | 5 × 10⁶ | 1.612 |
| Ingbritt | *Streptococcus mutans*/c | 5 × 10⁷ | >2.000 |
| P4 | *Streptococcus mutans*/e | 5 × 10² | 0.003 |
| P4 | *Streptococcus mutans*/e | 5 × 10³ | 0.012 |
| P4 | *Streptococcus mutans*/e | 5 × 10⁴ | 0.052 |
| P4 | *Streptococcus mutans*/e | 5 × 10⁵ | 0.473 |
| P4 | *Streptococcus mutans*/e | 5×10⁶ | 1.210 |
| P4 | *Streptococcus mutans*/e | 5×10⁷ | >2.000 |
| OMZ175 | *Streptococcus mutans*/f | 5×10² | 0.004 |
| OMZ175 | *Streptococcus mutans*/f | 5×10³ | 0.012 |
| OMZ175 | *Streptococcus mutans*/f | 5×10⁴ | 0.035 |
| OMZ175 | *Streptococcus mutans*/f | 5×10⁵ | 0.312 |
| OMZ175 | *Streptococcus mutans*/f | 5×10⁶ | 1.026 |
| OMZ175 | *Streptococcus mutans*/f | 5×10⁷ | 1.890 |
| Challis | *Streptococcus gordonii* | 5×10⁵ | 0.003 |
| Challis | *Streptococcus gordonii* | 5×10⁶ | 0.004 |
| Challis | *Streptococcus gordonii* | 5×10⁷ | 0.018 |
| IFO13956 | *Streptococcus salivarius* | 5×10⁵ | 0.004 |
| IFO13956 | *Streptococcus salivarius* | 5×10⁶ | 0.005 |
| IFO13956 | *Streptococcus salivarius* | 5×10⁷ | 0.007 |
| ATCC35037 | *Streptococcus oralis* | 5×10⁵ | 0.005 |
| ATCC35037 | *Streptococcus oralis* | 5×10⁶ | 0.007 |
| ATCC35037 | *Streptococcus oralis* | 5×10⁷ | 0.007 |
| ATCC15911 | *Streptococcus mitis* | 5×10⁵ | 0.004 |
| ATCC15911 | *Streptococcus mitis* | 5×10⁶ | 0.005 |
| ATCC15911 | *Streptococcus mitis* | 5×10⁷ | 0.007 |
| ATCC10556 | *Streptococcus sanguis* | 5×10⁵ | 0.006 |
| ATCC10556 | *Streptococcus sanguis* | 5×10⁶ | 0.006 |
| ATCC10556 | *Streptococcus sanguis* | 5×10⁷ | 0.014 |

As shown in Tables 3 to 5, the antigen level of *Streptococcus mutans* for which reaction was identical to that detected when other streptococci (5 x 10⁷ cells/well) were used as antigen, was 5 x 10⁴ cells/well or less. The result showed that the reactivities of the antibodies, M-1, M-2 and M-3, against *Streptococcus mutans* were respectively 1000-fold or more greater than the reactivities against other streptococci. Further, it was also shown that when protease-treated Ingbritt was at least either used as antigen for immunization or used for purification, an effect similar to that obtained when used for both purposes could be obtained. Furthermore, it was also shown that the reactivities against *Streptococcus mutans* of serotypes other than c were respectively more than half of the reactivities against serotype c.

### Comparative example 1 [Preparation of polyclonal antibody by absorption method, and evaluation of reactivity of antibody]

### (1) [Preparation and purification of antiserum against Streptococcus mutans]

The antiserum against *Streptococcus mutans* was prepared by a method similar to that of Example 1 (2).

According to the method of Example 1 (1), 30 ml each of cell suspensions containing respectively about 2 x 10¹² cells/ml Challis, IFO13956 or ATCC 35037 were prepared. Subsequently, the cell suspension and 0.5 ml of antiserum were mixed, followed by reaction at 4 °C for 60 minutes. The mixed solution was then centrifuged at 4000 x g for 5 minutes to collect supernatant. The supernatant was filtered using a 0.22 µm filter.

Next, the supernatant sample was added to a column charged with 1 ml of Protein A-Sepharose which had been previously equilibrated with PBS. After the column was washed with 5 ml of PBS, elution was performed using 5 ml of 0.1 M glycine-hydrochroride buffer (pH 3.0). Immediately after elution, 1M Tris-hydrochloride (pH 9.0) was added to adjust at pH 7.4. The eluted fraction of IgG was confirmed by measuring A₂₈₀. About 5 mg of IgG was collected from 0.5 ml of the antiserum.

### (2) [Evaluation by direct ELISA method of polyclonal antibody against Streptococcus mutans]

By a method similar to that of Example 1 (4), the reactivity of the polyclonal antibody against *Streptococcus mutans* which had been prepared by an absorption method in (1) above against each strain of cells were evaluated (Table 6).

**[Table 6]**

| Strain | Species/Serotype | Cell count (cells/well) | A₄₀₅ |
|---|---|---|---|
| Ingbritt | *Streptococcus mutans*/c | 5×10⁴ | 0.008 |
| Ingbritt | *Streptococcus mutans*/c | 5×10⁵ | 0.080 |
| Ingbritt | *Streptococcus mutans*/c | 5×10⁶ | 0.921 |
| Ingbritt | *Streptococcus mutans*/c | 5×10⁷ | 1.762 |
| P4 | *Streptococcus mutans*/e | 5×10⁴ | 0.005 |
| P4 | *Streptococcus mutans*/e | 5×10⁵ | 0.071 |
| P4 | *Streptococcus mutans*/e | 5×10⁶ | 0.581 |
| P4 | *Streptococcus mutans*/e | 5×10⁷ | 1.362 |
| OMZ175 | *Streptococcus mutans*/f | 5×10⁴ | 0.005 |
| OMZ175 | *Streptococcus mutans*/f | 5×10⁵ | 0.051 |
| OMZ175 | *Streptococcus mutans*/f | 5×10⁶ | 0.421 |
| OMZ175 | *Streptococcus mutans*/f | 5×10⁷ | 1.008 |
| Challis | *Streptococcus gordonii* | 5×10⁵ | 0.008 |
| Challis | *Streptococcus gordonii* | 5×10⁶ | 0.007 |
| Challis | *Streptococcus gordonii* | 5×10⁷ | 0.050 |
| IFO13956 | *Streptococcus salivarius* | 5×10⁵ | 0.007 |
| IFO13956 | *Streptococcus salivarius* | 5×10⁶ | 0.009 |
| IFO13956 | *Streptococcus salivarius* | 5×10⁷ | 0.010 |
| ATCC35037 | *Streptococcus oralis* | 5×10⁵ | 0.004 |
| ATCC35037 | *Streptococcus oralis* | 5×10⁶ | 0.010 |
| ATCC35037 | *Streptococcus oralis* | 5×10⁷ | 0.030 |
| ATCC15911 | *Streptococcus mitis* | 5×10⁵ | 0.003 |
| ATCC15911 | *Streptococcus mitis* | 5×10⁶ | 0.004 |
| ATCC15911 | *Streptococcus mitis* | 5×10⁷ | 0.010 |
| ATCC10556 | *Streptococcus sanguis* | 5×10⁵ | 0.006 |
| ATCC10556 | *Streptococcus sanguis* | 5×10⁶ | 0.005 |
| ATCC10556 | *Streptococcus sanguis* | 5×10⁷ | 0.015 |

As is clear from Table 6, the reactivity against *Streptococcus mutans* of the anti*-Streptococcus mutans* antibody purified by an absorption method of the related art was 100-fold or more but less than 1000-fold greater than its reactivity against *Streptococcus salivarius, Streptococcus mitis,* or *Streptococcus sanguis.*

### Example 2 [Preparation and evaluation of immunochromatography test strip for measuring Streptococcus mutans]

### (1) [Preparation of polyclonal antibody against Streptococcus mutans labeled with colloidal gold]

88 µl of 100 mM K₂CO₃ was added to 10 ml of a commercial solution of colloidal gold (EY Laboratory) with a colloidal particle size of 40 nm, pH was adjusted at 9.0, and then the solution was treated with a 0.22 µm filter. Absorbance of the colloidal gold solution at 520 nm was A₅₂₀=1.0 when measured.

Subsequently, 64 µl of 2 mM borate buffer (pH 9.0) of M-2 adjusted at 1 mg/ml was added to the above colloidal gold solution while stirring, and then the mixed solution was allowed to stand at room temperature for 5 minutes. Next, 1.1 ml of 10% skim milk-2mM borate buffer (pH 9.0) was added (final concentration of skim milk was 1%) while stirring, and the mixed solution was then allowed to stand at room temperature for 30 minutes. Subsequently, the reaction solution was centrifuged at 10,000 x g at 10°C for 30 minutes to remove supernatant. Then, 2 ml of 2 mM PBS (pH 7.4) was added, and the colloidal gold fraction of the lower layer was then re-suspended. The absorbance at 520 nm of the re-suspended fraction was A₅₂₀=4.9 when measured. The thus obtained colloidal gold fraction (hereinafter, also denoted as "colloidal gold-labeled M-2") was stored at 4°C.

### (2) [Preparation of immunochromatography test strip]

On the detection line 6 and control determination line 7 which are located on the development membrane 4 comprising nitrocellulose membrane (MILLIPORE, Hi-Flow Plus Membrane, HF 75, 25 mm x 6 mm) as shown in Figs. 1 and 2, 1 mg/ml M-3 and 1 µl of anti-rabbit IgG (H+L) polyclonal antibody were spotted, respectively, followed by drying at 37°C for 60 minutes within an incubator to immobilize the antibodies. The antibody-immobilized membrane was shaken in a 1% skim milk-0.1% Triton X 100 aqueous solution at room temperature for 5 minutes. After the membrane was shaken in 10 mM phosphate buffer (pH 7.4) at room temperature for 10 minutes, the membrane was taken out and then dried in a desiccator for 60 minutes while sucking with a vacuum pump.

In addition, after the conjugate pad 3 (MILLIPORE, 7.5 mm x 6 mm) was shaken in a 0.5% PVA-0.5 % sucrose aqueous solution for 1 minute, the pad was taken out and then dried in a desiccator for 60 minutes while sucking with a vacuum pump. 25 µl of the colloidal gold-labeled M-2 adjusted at A₅₂₀=1.0 was added to the conjugate pad, and then the pad was dried in a desiccator for 60 minutes while sucking with a vacuum pump. Furthermore, after the sample pad 2 (MILLIPORE, 17 mm x 6 mm) was shaken in a 1% Tween 20-PBS aqueous solution for 1 minute, the pad was taken out and then dried in a desiccator for 60 minutes while sucking with a vacuum pump. In addition, the absorbent pad 5 (MILLIPORE, 20 mm x 6 mm) was used untreated.

The thus prepared respective components of an immunochromatography test strip as shown in Fig. 1 were disposed on a plastic support, so that an immunochromatography test strip as shown in Fig. 2 was assembled.

### (3) [Evaluation of specificity and sensitivity of immunochromatography test strip]

The cell suspension prepared in Example 1 (1) was appropriately diluted with PBS to prepare a test solution. 100 µl of the test solution was added onto the sample pad 2 of the immunochromatography test strip. 10 minutes later, the presence or absence of spots was determined. Specifically, determination was performed by visually identifying the degree of colloidal gold captured on the spot of the antibody for detection based on the 4-stage criteria shown in the above Table 1 (+++: strongly positive, ++: positive, +: weakly positive, -: negative) thereby evaluating sensitivity and quantitativity. Table 7 shows the results. While no coloration was observed on the determination line for other streptococci even when the concentration was 10⁸ cells/ml, 10⁵ cells/ml *Streptococcus mutans* could be specifically detected.

**[Table 7]**

| Strain | Species/Serotype | Cell concentration in test solution (cells/ml) | Determination |
|---|---|---|---|
| Ingbritt | *Streptococcus mutans*/c | 10⁷ | +++ |
| Ingbritt | *Streptococcus mutans*/c | 10⁶ | ++ |
| Ingbritt | *Streptococcus mutans*/c | 10⁵ | + |
| Ingbritt | *Streptococcus mutans*/c | 10⁴ | - |
| P4 | *Streptococcus mutans*/e | 10⁷ | +++ |
| P4 | *Streptococcus mutans*/e | 10⁶ | ++ |
| P4 | *Streptococcus mutans*/e | 10⁵ | + |
| P4 | *Streptococcus mutans*/e | 10⁴ | - |
| OMZ175 | *Streptococcus mutans*/f | 10⁷ | +++ |
| OMZ175 | *Streptococcus mutans*/f | 10⁶ | ++ |
| OMZ175 | *Streptococcus mutans*/f | 10⁵ | + |
| OMZ175 | *Streptococcus mutans*/f | 10⁴ | - |
| Challis | *Streptococcus gordonii* | 10⁸ | - |
| OMZ175 | *Streptococcus mutans*/f | 10⁸ | - |
| IFO13956 | *Streptococcus salivarius* | 10⁸ | - |
| ATCC35037 | *Streptococcus oralis* | 10⁸ | - |
| ATCC15911 | *Streptococcus mitis* | 10⁸ | - |
| ATCC10556 | *Streptococcus sanguis* | 10⁸ | - |

### Comparative example 2 [Evaluation of immunochromatography test strip using polyclonal antibody against Streptococcus mutans purified by absorption method]

By a method similar to that described in Example 2 (1) and (2) except that the polyclonal antibody against *Streptococcus mutans* prepared in Comparative example 1 was used instead of M-2 or M-3, an immunochromatography test strip was prepared. The strip was evaluated for its sensitivity and reactivity against cells according to the method described in Example 2 (3). Table 8 shows the results.

**[Table 8]**

| Strain | Species/Serotype | Cell concentration in test solution (cells/ml) | Determination |
|---|---|---|---|
| Ingbritt | *Streptococcus mutans*/c | 10⁷ | +++ |
| Ingbritt | *Streptococcus mutans*/c | 10⁶ | ++ |
| Ingbritt | *Streptococcus mutans*/c | 105 | - |
| Ingbritt | *Streptococcus mutans*/c | 104 | - |
| P4 | *Streptococcus mutans*/e | 10⁷ | +++ |
| P4 | *Streptococcus mutans*/e | 10⁶ | ++ |
| P4 | *Streptococcus mutans*/e | 10⁵ | - |
| P4 | *Streptococcus mutans*/e | 10⁴ | - |
| OMZ175 | *Streptococcus mutans*/f | 10⁷ | +++ |
| OMZ175 | *Streptococcus mutans*/f | 10⁶ | + |
| OMZ175 | *Streptococcus mutans*/f | 10⁵ | - |
| OMZ175 | *Streptococcus mutans*/f | 10⁴ | - |
| Challis | *Streptococcus gordonii* | 10⁸ | - |
| OMZ175 | *Streptococcus mutans*/f | 10⁸ | - |
| IFO1395 | *Streptococcus salivarius* | 10⁸ | + |
| ATCC35037 | *Streptococcus oralis* | 10⁸ | - |
| ATCC15911 | *Streptococcus mitis* | 10⁸ | - |
| ATCC10556 | *Streptococcus sanguis* | 10⁸ | - |

As shown in Table 8, 10⁵ cells/ml *Streptococcus mutans* could not be detected when the polyclonal antibody purified by an absorption method of the related art was used.

This is because antibodies specifically reacting with *Streptococcus mutans* were removed by the absorption treatment together with antibodies that cause cross-reaction. Accordingly, when polyclonal antibodies produced by the absorption treatment were used, *Streptococcus mutans* at a concentration of about 10⁵ cells/ml could not be accurately measured when oral streptococci other than mutans streptococci, such as *Streptococcus gordonii, Streptococcus salivarius, Streptococcus oralis, Streptococcus mitis* or *Streptococcus sanguis*, coexisted in the order of 10⁸ cells/ml in a test solution.

### Example 3 [Detection of Streptococcus mutans from dental plaque by immunochromatography test strip]

### (1) [Preparation of test solution containing dental plaque]

A subject was asked to chew a paraffin pellet for about 1 minute, and secreted saliva was collected. The saliva was ultrasonicated for 20 seconds at 60 W, thereby obtaining a test solution containing dental plaque.

### (2) [Quantitative determination of Streptococcus mutans in solution containing dental plaque sample by culture method]

The original test solution containing dental plaque obtained according to the method of (1) above was appropriately diluted. 100 µl of the diluted solution was added onto Mitis Salivarius Bacitracin (hereinafter, also referred to as "MSB") solid media and BHI solid media, followed by culturing at 37°C for 24 to 48 hours under anaerobic conditions. The number of colonies formed on MSB and BHI solid media was counted, and then the concentration of mutans streptococci was calculated as cells/ml based on the dilution ratio of the test solution.

The colonies of *Streptococcus mutans* on MSB and BHI media was morphologically identified. When colonies were morphologically indistinguishable, they were subjected to pure culturing and then to an immunoassay using antibodies serotype-specific to mutans streptococci, and a biochemical method, such as a sugar fermentation test, thereby identifying *Streptococcus mutans.*

### (3) [Measurement of Streptococcus mutans in test solution containing dental plaque by immunochromatography test strip]

*Streptococcus mutans* in a sample to be tested containing dental plaque was measured using the immunochromatography test strip prepared by a method similar to that of Example 2 (2). The coloration intensity of a spot at 10 minutes after adding the test solution on the immunochromatography test strip was classified based on the criteria shown in Table 1 above. The results of identification and classification into the 4 stages (+++: strongly positive, ++: positive, +: weakly positive, -: negative) and the cell count of *Streptococcus mutans* obtained by the culture method of (2) were compared. Table 9 shows the results.

**[Table 9]**

| | Culture method | Immunochromatography |
|---|---|---|
| Sample | *Streptococcus mutans* (x 10⁵ cells/ml) | Determination |
| 1 | 139.0 | +++ |
| 2 | 122.0 | +++ |
| 3 | 52.8 | ++ |
| 4 | 29.0 | ++ |
| 5 | 18.0 | ++ |
| 6 | 16.0 | ++ |
| 7 | 8.0 | - |
| 8 | 7.0 | - |
| 9 | 3.92 | - |
| 10 | 1.52 | - |
| 11 | 0.88 | - |
| 12 | 0.78 | - |
| 13 | 0.01 | - |
| 14 | 0.00 | - |

As shown in Table 9, the coloration intensity of a spot correlating with the concentration of *Streptococcus mutans* obtained by the culture method was obtained. 10⁶ cells/ml or more *Streptococcus mutans* in a test solution could be specifically detected depending on concentration using the immunochromatography test strip of the present invention.

### Example 4 [Measurement of Streptococcus mutans by immunochromatography test strip using antigen extract from dental plaque as test solution]

### (1) [Preparation of antigen extract]

100 µl of saliva collected in Example 3 (1) was centrifuged to remove supernatant. The precipitate was suspended in 500 µl of 0.1 M NaOH, and then the suspension was centrifuged to remove supernatant. Next, the precipitate was suspended in 500 µl of PBS, and then the suspension was centrifuged to remove supernatant. After a similar procedure using PBS was performed again, 10 µl of 4 M acetic acid and 5 µl of 2 M sodium nitrite were added to the precipitate, and mixed well, and then the mixture was allowed to stand at room temperature for 10 minutes. 45 µl of 1M Tris (pH unadjusted) containing 0.05% Tween 20 and 40 µl of 0.1 M phosphate buffer (pH 7.5) containing 0.05% Tween 20 were added, so that an antigen extract was prepared.

### (2) [Measurement of Streptococcus mutans by immunochromatography test strip using antigen extract as test solution]

By using an immunochromatography test strip prepared by a method similar to that of Example 2 (2) except that a nitrocellulose membrane to be used for a test strip was Hi-Flow Plus Membrane, HF180, manufactured by MILLIPORE, *Streptococcus mutans* was measured using the antigen extract prepared in (1) above as a test solution. The coloration intensity of a spot at 10 minutes after adding the test solution on the immunochromatography test strip was classified based on the criteria shown in Table 1 above. The results of identification and classification into the 4 stages (+++: strongly positive, ++: positive, +: weakly positive, -: negative) and the cell count of *Streptococcus mutans* obtained by the culture method of Example 3 (2) were compared. Table 10 shows the results.

**[Table 10]**

| | Culture method | Immunochromatography |
|---|---|---|
| Sample | *Streptococcus mutans* (x 10⁵ cells/ml) | Determination |
| 1 | 139.0 | +++ |
| 2 | 122.0 | +++ |
| 3 | 52.8 | +++ |
| 4 | 29.0 | ++ |
| 5 | 18.0 | ++ |
| 6 | 16.0 | ++ |
| 7 | 8.0 | + |
| 8 | 7.0 | + |
| 9 | 3.92 | + |
| 10 | 1.52 | + |
| 11 | 0.88 | + |
| 12 | 0.78 | - |
| 13 | 0.01 | - |
| 14 | 0.00 | - |

As shown in Table 10, the coloration intensity of the spot correlating with concentration of *Streptococcus mutans* obtained by the culture method was obtained. 10⁵ cells/ml *Streptococcus mutans* was detected by extraction treatment.

### [Effect of the Invention]

The use of the polyclonal antibody according to the present invention enables highly sensitive and direct measurement of *Streptococcus mutans* without culturing a test solution containing saliva or dental plaque wherein other streptococci co-exist with *Streptococcus mutans.* According to the present invention, a novel diagnosis system of the risk of dental caries can be established.

## Claims

1. A polycional antibody, whose reactivity against *Streptococcus mutans* is 1000-fold or more greater than its reactivity against each of *Streptococcus gordonii, Streptococcus salivarius, Streptococcus oralis*, *Streptococcus mitis* and *Streptococcus sanguis.*

2. The polyclonal antibody of claim 1, which is obtained by immunizing an immune animal with the whole cells of *Streptococcus mutans* treated with protease, or the antigen extract thereof.

3. The polyclonal antibody of claim 1, which is purified by affinity purification using the whole cells of *Streptococcus mutans* treated with protease, or the antigen extract thereof.

4. A method for producing a polyclonal antibody of claim 1 which is obtained by immunizing an immune animal with the whole cells of *Streptococcus mutans* treated with protease, or the antigen extract thereof.

5. A method for producing a polyclonal antibody of claim 1, which comprises obtaining antibodies as raw material by immunizing an immune animal with at least one type of substance selected from the group consisting of the whole cells of *Streptococcus mutans* treated with protease, the antigen extract thereof, the whole cells of *Streptococcus mutans* and the antigen extract thereof; and then purifying from the antibodies as raw material by affinity purification using the whole cells of *Streptococcus mutans* treated with protease or the antigen extract thereof.

6. A method for measuring *Streptococcus mutans*, which comprises measuring *Streptococcus mutans* contained in a test solution by an immunoassay using the polyclonal antibody of any one of claims 1 to 3.

7. The method for measuring *Streptococcus mutans* of claim 6, wherein the immunoassay is an immunochromatography method.

8. The method for measuring the antigen extract obtained from *Streptococcus mutans*, which comprises measuring the antigen extract obtained from *Streptococcus mutans* contained in a test solution by an immumoassay using the polyclonal antibody of any one of claims 1 to 3.

9. An immunoassay reagent, which comprises the polyclonal antibody of any one of claims 1 to 3.

10. An immunochromatography test strip, which comprises: a sample pad for absorbing and retaining temporarily a test solution, a conjugate pad for retaining temporarily a labeled antibody, and a development membrane wherein antibodies for detection are immobilized and the test solution temporarily absorbed and retained by the sample pad and the labeled antibodies that flow from the above conjugate pad accompanying the test solution are spread: and wherein these components are joined in this order, and one of or both the labeled antibody and the antibody for detection are the polyclonal antibodies of any one of claims 1 to 3.
